# EUROPEAN PATENT SPECIFICATION

(11) **EP 3 666 169 B1**
(45) Date of publication and mention of the grant of the patent: **29.10.2025**
(21) Application number: 18211490.0
(22) Date of filing: 11.12.2018
(51) Int. Cl.: G06F 3/00, G06V 40/00

(54) **DRIVER MONITORING SYSTEM**
FAHRERÜBERWACHUNGSSYSTEM
SYSTÈME DE SURVEILLANCE DE CONDUCTEUR

(43) Date of publication of application: 17.06.2020
(73) Proprietor: Aptiv Technologies AG, 8200 Schaffhausen (CH)
(72) Inventor: BOHDAN, Lukasz, 47-330 Zdzieszowice (PL); MUCHA, Rafal, 31-845 KRAKOW (PL)
(74) Representative: Manitz Finsterwald Patent- und Rechtsanwaltspartnerschaft mbB

(56) References cited:
- EP-A1- 2 918 225
- US-A1- 2005 100 191
- US-A1- 2013 188 834
- US-A1- 2016 170 486

## Description

### TECHNICAL FIELD OF THE INVENTION

The invention relates to a driver monitoring system of a motor vehicle. More particularly the invention relates a driver monitoring system based on dark pupil tracking method.

### BACKGROUND OF THE INVENTION

Driver monitoring systems are already well known in the automotive domain as for detecting driver drowsiness. In general for enhanced safety function of a car, tracking pupils of a driver is needed. Solutions for tracking pupils are generally based on a camera pointing the face of the driver. Implementation of camera for supervising the eyes of a driver is a challenge regarding style of the body compartment of the motor vehicle, performance of capturing eyes position and cost.

It is therefore important to propose a new solution to solve these problems.

US 2016/170486 A1 discloses a device for acquiring a combined eye gaze image of an object under dark-eye effect conditions, with a first camera, a second camera, a first light source and a second light source being located on opposite sides of, and on essentially equal distance to a central optical axis. A control unit is arranged to acquire the combined eye gaze image by capturing, in a first point in time, a first frame of the object with the first camera with the second light source activated, and, at a second point in time, capturing a second frame of the object with the second camera with the first light source activated.

### SUMMARY OF THE INVENTION

A driver monitoring system for a motor vehicle configured to track pupils of a driver of the motor vehicle comprises a first camera device including a first main optical axis, said first camera device being configured to capture a first set of images of the pupils of the drive; a first illumination device including a first main light axis, said first illumination device being configured to illuminate the pupils of the driver during a capture operation of the first set of images; a second camera device including a second main optical axis, said second camera device being configured to capture a second set of images of the pupils of the driver; a second illumination device including a second main light axis, said second illumination device being configured to illuminate the pupils of the driver during a capture operation of the second set of images; a first camera assembly comprising the first camera device and the second illumination device, wherein the first camera device is physically assembled with the second illumination device; a second camera assembly comprising the second camera device and the first illumination device, wherein the second camera device is physically assembled with the first illumination device.

The system may further comprise an electronic control unit in electrical communication with the first camera assembly and with the second camera assembly, said electronic control unit comprising switching means for enabling together the first camera device and the first illumination device while disabling together the second camera device and the second illumination device. The first illumination device and the second illumination device may comprise each at least one infra-red light source. The first camera assembly and the second camera assembly may be both type of infra-red camera.

The first camera device and the second illumination device may be arranged from each other at a first distance less than 40 millimeters, preferably less than 35 millimeters. The second camera device and the first illumination device may be arranged from each other at a second distance less than 40 millimeters, preferably less than 35 millimeters. The first main optical axis and the first main light axis may intersect at a first angle greater than 5°, preferably greater than 10°. The second main optical axis and the second main light axis may intersect at a second angle greater than 5°, preferably greater than 10°.

According to the invention, a vehicle driver compartment comprises the driver monitoring system described above, said vehicle driver compartment comprises a front panel instrument assembly comprising a dashboard portion facing the vehicle's driver seat wherein the first camera assembly and the second camera assembly are arranged with the front panel instrument; the first main optical axis and the first main light axis intersect at the driver's head zone of the driver's seat; the second main optical axis and the second main light axis intersect at the driver's head zone of the driver's seat.

The first camera assembly may be arranged within the dashboard portion.

According to the invention, a method to operate the driver monitoring system for a motor vehicle described above, comprises the steps of:
providing a first camera assembly comprising a first camera device and a second illumination device;
providing a second camera assembly comprising a second camera device and a first illumination device;
capturing a first set of images of the pupils of the driver with the first camera device while illuminating the pupils of the driver by the first illumination device.

The method may further comprise the step of :
capturing a second set of images of the pupils of the driver with the second camera device while illuminating the pupils of the driver by the second illumination device.

The method may further comprise the step of:
enabling together the first camera device and the first illumination device while disabling together the second camera device and the second illumination device.

The method may further comprise the step of:
enabling together the second camera device and the second illumination device while disabling together the first camera device and the first illumination device.

The method may further comprise the steps of:
arranging the first camera assembly and the second camera assembly with the front panel instrument of the driver compartment of the motor vehicle;
orienting the first main optical axis and the first main light axis such that the first main optical axis and the first main light axis globally intersect at the driver's face;
orienting the second main optical axis and the second main light axis such that the second main optical axis and the second main light axis globally intersect at the driver's face.

The method may further comprise the step of:
orienting the first main optical axis and the first main light axis such that the first main optical axis and the first main light axis intersect at a first angle greater than 5°, preferably greater than 10°.

The method may further comprise the step of:
orienting the second main optical axis and the second main light axis, such that the second main optical axis and the second main light axis intersect at a second angle greater than 5°, preferably greater than 10°.

The method may further comprise the step of:
providing the first camera device and the second illumination device distanced by a first distance less than 40 millimeters, preferably less than 35 millimeters.

The method may further comprises the step of:
providing the second camera device and the first illumination device distanced by a second distance less than 40 millimeters, preferably less than 35 millimeters.

### BRIEF DESCRIPTION OF THE DRAWING

Other features, objects and advantages of the invention will become apparent from reading the detailed description that follows, and the attached drawing, given by way of example and in which:
- Figure 1 is a schematic diagram of a driver monitoring system according to one embodiment of the invention.
- Figure 2 is a flow chart of a method to operate the driver monitoring system according to one embodiment of the invention.
- Figure 3 is a flow chart example of the method to arranged camera device and illumination device according to the flow chart of figure 2.
- Figure 4 is a flow chart example of the method to capture pupils images according to the flow chart of figure 2.

### DETAILED DESCRIPTION OF THE PREFFERED EMBODIMENTS

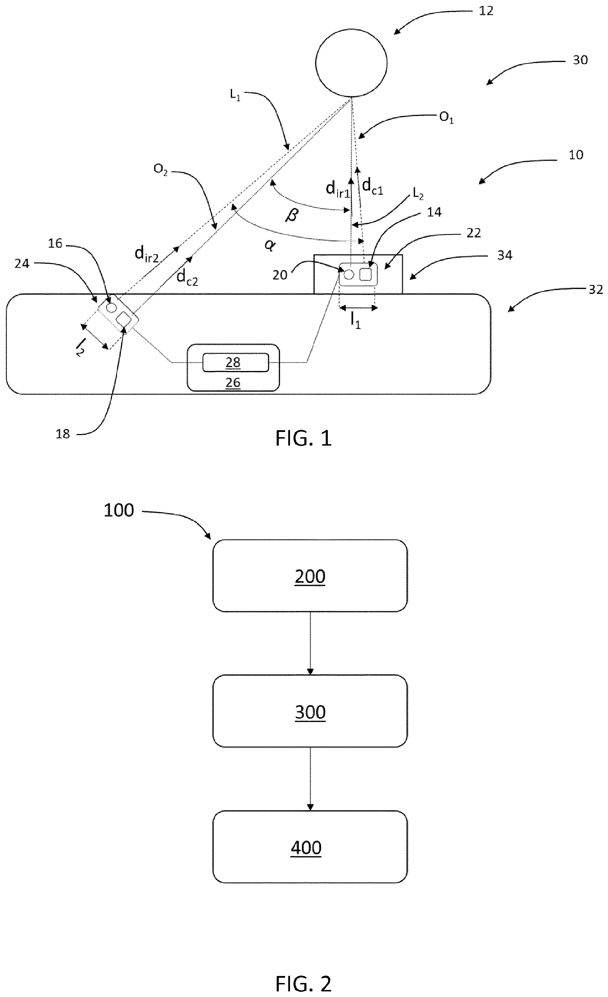

According to figure 1, a driver monitoring system 10 for a motor vehicle configured to track pupils of a driver 12 of the motor vehicle is shown. The circle, reference number 12, represents the face of a driver facing the front panel instrument assembly 32 of the motor vehicle, more particularly, facing the portion of the dashboard 34 wherein gauge meter as speedometer may be arranged.

The system 10 comprises a first camera device 14 including a first main optical axis O₁. The first main optical axis O₁, or first camera optical axis, is the center line of the field of view of said first camera device 14. The first camera device is configured to capture a first set of images of the pupils of the driver 12, in order, for example, to be able to determine a movement of the pupils and also the gaze direction of the eyes.

The system 10 further comprises a first illumination device 16 including a first main light axis L₁. The first main light axis L₁, or first light source optical axis, is the center line of the illumination pattern of the first illumination device 16. The first illumination device 16 is configured to illuminate the pupils of the driver 12 during a capture operation of the first set of images.

In order to get reliable information on pupils of the driver 12 of the motor vehicle, redundancy of means to capture set of images of the pupils of the driver 12 is needed such that the driver monitoring system 10 may cross images acquisition by both means to capture set of images. Thus, the system further comprises a second camera device 18 including a second main optical axis O₂. The second main optical axis O₂, or second camera optical axis, is the center line of the field of view of said second camera device 18. The second camera device is configured to capture a second set of images of the pupils of the driver 12.

The system 10 further comprise a second illumination device 20 including a second main light axis L₂. The second main light axis L₂, or second light source optical axis, is the center line of the illumination pattern of the second illumination device 20. The second illumination device 20 is configured to illuminate the pupils of the driver 12 during a capture operation of the second set of images.

To provide camera devices and illumination devices that make the integration of the driver monitoring system 10 easily, camera devices and illuminations devices should be provided as two camera assembly comprising each a camera device and an illumination device. In addition, for dark pupil tracking method, angulation between a camera device and its associated illumination device is an issue. Dark pupil tracking requires higher angulation between camera device and its associated illumination device than a bright pupil tracking method. An associated illumination device with a camera device is the illumination device that illuminates driver face during the camera device operation. Thus, the camera device and its associated illumination device have to be far enough from each other.

According to the invention, the driver monitoring system 10 comprises a first camera assembly 22 comprising the first camera device 14 and the second illumination device 20, and a second camera assembly 24 comprising the second camera device 18 and the first illumination device 16. As such, the first camera device 14 is physically assembled with the second illumination device 20, but the first camera device 14 is operationally associated with the first illumination device 16. As such, the second camera device 18 is physically assembled with the first illumination device 16, but the second camera device 18 is operationally associated with the second illumination device 20.

The driver monitoring system 10 further comprises an electronic control unit 26 in electrical communication with the first camera assembly 22 and with the second camera assembly 24. The electronic control unit 26 is configured to synchronize the behavior of the first camera device 14 with the first illumination device 16. The electronic control unit 26 is also configured to synchronize the behavior of the second camera device 18 with the second illumination device 20.

During the first set of images acquisition operation of the first camera device 14 of the first camera assembly 22, the electronic control unit 26 enables the first illumination device 16 of the second camera assembly 24 to illuminate or flash the pupils of a driver 12 of the motor vehicle.

During the second set of images acquisition operation of the second camera device 18 of the second camera assembly 24, the electronic control unit 26 enables the second illumination device 20 of the first camera assembly 22 to illuminate or flash the pupils of a driver 12 of the motor vehicle.

As such, the electronic control unit 26 comprises switching means 28 for enabling together the first camera device 14 and the first illumination device 16 while disabling together the second camera device 18 and the second illumination device 20. The electronic control unit 26 may be a microcontroller wherein the switching means are digital means providing data communication between the microcontroller and both first camera assembly 22 and second camera assembly 24.

To avoid too much disturbances of the driver of the motor vehicle, the first illumination device 16 and the second illumination device 20 comprise each at least one infra-red light source that is activated during capture operation of a set of images of the pupils of the driver 12. Preferably, the first camera assembly 22 and the second camera assembly 24 are both type of infra-red camera such that camera packaging assembly is minimized. Generally, to get acceptable camera assembly dimension according to the constraint of the vehicle's cabin design, the first camera device 14 and the second illumination device 20 are arranged from each other at a first distance l₁ less than 40 millimeters, preferably less than 35 millimeters, especially when said first camera assembly is arranged within the dashboard potion 34. Additionally, the second camera device 18 and the first illumination device 16 are arranged from each other at a second distance l₂ less than 40 millimeters, preferably less than 35 millimeters.

In order to provide a reliable dark pupil tracking method, according to a general distance between the front panel instrument assembly 32 and the pupils of the driver 12 of the motor vehicle, it is recommended that the first main optical axis O₁ and the first main light axis L₁ intersect at a first angle α greater than 5°, even preferably greater than 10°.

According to a cross operation of capturing set of images of the pupils of the driver, the second main optical axis O₂ and the second main light axis L₂ intersect at a second angle β greater than 5°, even preferably greater than 10°.

According to figure 1, a vehicle driver compartment 30 comprises the driver monitoring system 10 is shown. The vehicle driver compartment 30 comprises the front panel instrument assembly 32 including the dashboard portion 34 facing the vehicle's driver seat (not shown) wherein stands the driver 12.

The first camera assembly 22 and the second camera assembly 24 are arranged with the front panel instrument 32. Both may be integrated inside the front panel assembly, not visible by the driver 12, such that both camera assembly 22, 24 are placed behind and opaque area of the front panel instrument 32 in order to be able to capture sets of images of the pupils of the driver 12 of the motor vehicle.

Arrangement with the front panel assembly 32 is such that the first main optical axis O₁ and the first main light axis L₁ intersect at the driver's head zone of the driver's seat; and the second main optical axis O₂ and the second main light axis L₂ intersect at the driver's head zone of the driver's seat. More particularly, the first camera assembly 22 is arranged with the dashboard portion 34.

The first camera device 14, part of the first camera assembly 22, is arranged such that its first main optical axis O₁ is pointing in a first optical direction d_{c1} toward the face of the driver 12 of the motor vehicle. The first illumination device 16, part of the second camera assembly 24, is arranged such that its first main light axis L₁ is pointing in a second light direction dᵢᵣ₂ toward the face of the driver 12 of the motor vehicle. The second camera device 18, part of the second camera assembly 24, is arranged such that its second main optical axis O₂ is pointing in a second optical direction d_{c2} toward the face of the driver 12 of the motor vehicle. The second illumination device 20, part of the first camera assembly 22, is arranged such that its second main light axis L₂ is pointing in a first light direction dᵢᵣ₁ toward the face of the driver 12 of the motor vehicle.

According the figure 2, a method 100 to operate the driver monitoring system 10 for a motor vehicle as described at figure 1 is shown. The method comprises three main steps. At first, the method comprises a first step of providing 200 a first camera assembly 22 comprising a first camera device 14 and a second illumination device 20, and a second step of providing 300 a second camera assembly 24 comprising a second camera device 18 and a first illumination device 16. Then the method 100 comprises a third step of capturing 400 set of images of the pupils of the driver 12 by means of the first camera assembly 22 and of the second camera assembly 24.

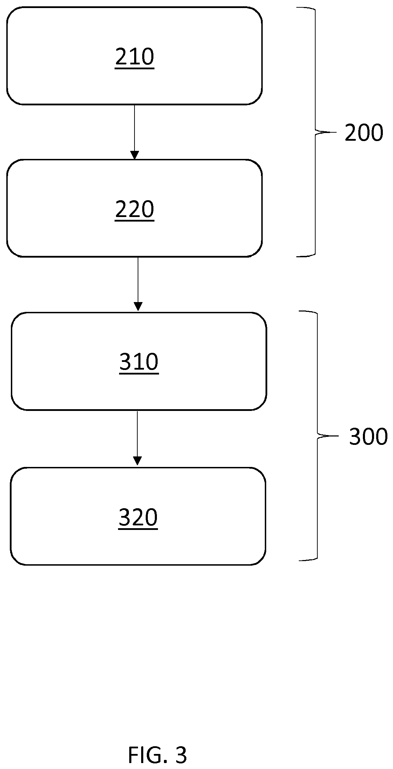

According to figure 3, the first step of providing 200 the first camera assembly 22 comprising a step of providing 210 the first camera device 14 and the second illumination device 20 distanced by a first distance l₁ less than 40 millimeters, preferably less than 35 millimeters; and a step of assembling 220 the first camera device 14 and the second illumination device 20 such that the first distance l₁ is less than 40 millimeters, preferably less than 35 millimeters. An infra-red type of camera assembly may suit with the above steps.

According to figure 3, the second step of providing 300 the second camera assembly 24 comprising a step of providing 310 the second camera device 18 and the first illumination device 16 distanced by a second distance l₂ less than 40 millimeters, preferably less than 35 millimeters; and a step of assembling 320 the second camera device 18 and the first illumination device 16 such that the second distance l₂ is less than 40 millimeters, preferably less than 35 millimeters. An infra-red type of camera assembly may suit with the above steps.

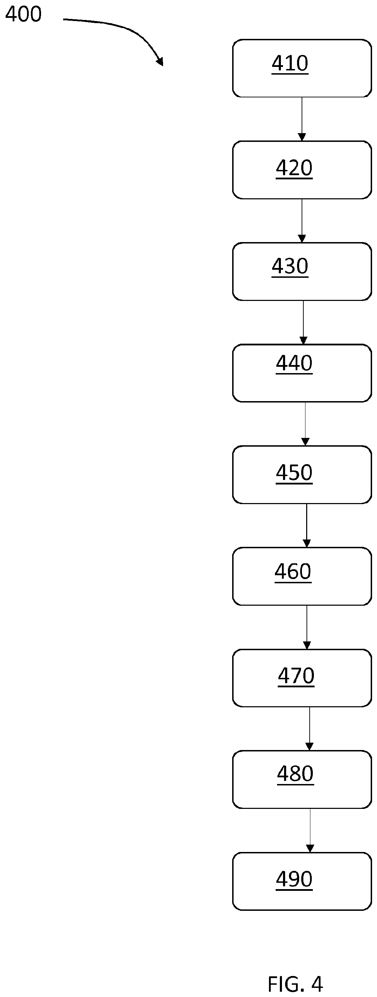

According to figure 4, the step of capturing 400 set of images of the pupils of the driver 12 comprises several step. A firs step comprises a step of arranging 410 the first camera assembly 22 and the second camera assembly 24 with the front panel instrument 32 of the driver compartment 30 of the motor vehicle. More particularly, the first camera assembly 22 is arranged within the dashboard portion 34 of the front panel instrument 32 wherein gauge meter as speedometer is located. Then another step comprises a step of orienting 420 the first main optical axis O₁ and the first main light axis L₁ such that the first main optical axis O₁ and the first main light axis L₁ globally intersect at the driver's face. In addition, for redundancy operation, another step comprises a step of orienting 430 the second main optical axis O₂ and the second main light axis L₂ such that the second main optical axis O₂ and the second main light axis L₂ globally intersect at the driver's face.

To get an efficient dark pupil tracking method, the step of capturing 400 set of images of the pupils of the driver 12 comprising a step of orienting 440 the first main optical axis O₁ and the first main light axis L₁ such that the first main optical axis O₁ and the first main light axis L₁ intersect at a first angle α greater than 5°, preferably greater than 10°. In addition, the step of capturing 400 set of images of the pupils of the driver 12 comprising a step of orienting 450 the second main optical axis O₂ and the second main light axis L₂, such that the second main optical axis O₂ and the second main light axis L₂ intersect at a second angle β greater than 5°, preferably greater than 10°.

Then for cross operating method, the step of capturing 400 set of images of the pupils of the driver 12 comprising a step of enabling 460 together the first camera device 14 and the first illumination device 16 while disabling together the second camera device 18 and the second illumination device 20, and then the step of capturing 400 set of images of the pupils of the driver 12 comprises a step of capturing 470 a first set of images of the pupils of the driver 12 with the first camera device 14 while illuminating the pupils of the driver 12 by the first illumination device 16.

In addition, following said two previous steps, the step of capturing 400 set of images of the pupils of the driver 12 comprising a step of enabling 480 together the second camera device 18 and the second illumination device 20 while disabling together the first camera device 14 and the first illumination device 16, and then the step of capturing 400 set of images of the pupils of the driver 12 comprises a step of capturing 490 a second set of images of the pupils of the driver 12 with the second camera device 18 while illuminating the pupils of the driver 12 by the second illumination device 20.

## Claims

1. Driver monitoring system (10) for a motor vehicle configured to track pupils of a driver (12) of the motor vehicle, said system (10) comprising
a first camera device (14) including a first main optical axis (O₁), said first camera device (14) being configured to capture a first set of images of the pupils of the driver (12);
a first illumination device (16) including a first main light axis (L₁), said first illumination device (16) being configured to illuminate the pupils of the driver (12) during a capture operation of the first set of images;
a second camera device (18) including a second main optical axis (O₂), said second camera device (18) being configured to capture a second set of images of the pupils of the driver (12);
a second illumination device (20) including a second main light axis (L₂), said second illumination device (20) being configured to illuminate the pupils of the driver (12) during a capture operation of the second set of images;
**characterized in that** the system (10) further comprising
a first camera assembly (22) comprising the first camera device (14) and the second illumination device (20), wherein the first camera device (14) is physically assembled with the second illumination device (20);
a second camera assembly (24) comprising the second camera device (18) and the first illumination device (16), wherein the second camera device (18) is physically assembled with the first illumination device (16).

2. System (10) according to claim 1 characterized the system (10) further comprises an electronic control unit (26) in electrical communication with the first camera assembly (22) and with the second camera assembly (24), said electronic control unit (26) comprising switching means (28) for enabling together the first camera device (14) and the first illumination device (16) while disabling together the second camera device (18) and the second illumination device (20).

3. System (10) according to any one of the preceding claims **characterized in that** the first camera device (14) and the second illumination device (20) are arranged from each other at a first distance (l₁) less than 40 millimeters, preferably less than 35 millimeters.

4. System (10) according to claim 3 **characterized in that** the second camera device (18) and the first illumination device (16) are arranged from each other at a second distance (l₂) less than 40 millimeters, preferably less than 35 millimeters.

5. System (10) according to any one of the preceding claims **characterized in that** the first main optical axis (O₁) and the first main light axis (L₁) intersect at a first angle (α) greater than 5°, preferably greater than 10°; and the second main optical axis (O₂) and the second main light axis (L₂) intersect at a second angle (β) greater than 5°, preferably greater than 10°.

6. Vehicle driver compartment (30) comprising the system (10) of any one of the preceding claims, said vehicle driver compartment (30) comprising
a front panel instrument assembly (32) comprising a dashboard portion (34) facing the vehicle's driver seat,
**characterized in that**
the first camera assembly (22) and the second camera assembly (24) are arranged with the front panel instrument (32);
the first main optical axis (O₁) and the first main light axis (L₁) intersect at the driver's head zone of the driver's seat;
the second main optical axis (O₂) and the second main light axis (L₂) intersect at the driver's head zone of the driver's seat.

7. Vehicle driver compartment (30) according to claim 6 **characterized in that** the first camera assembly (22) is arranged within the dashboard portion (34).

8. Method (100) to operate the driver monitoring system (10) for a motor vehicle according to any one of the claims 1 to 5, said method (100) comprising the steps of:
providing (200) a first camera assembly (22) comprising a first camera device (14) and a second illumination device (20);
providing (300) a second camera assembly (24) comprising a second camera device (18) and a first illumination device (16);
capturing (470) a first set of images of the pupils of the driver (12) with the first camera device (14) while illuminating the pupils of the driver (12) by the first illumination device (16).

9. Method (100) according to claim 8, **characterized in that** the method (100) further comprises the step of :
capturing (490) a second set of images of the pupils of the driver (12) with the second camera device (18) while illuminating the pupils of the driver (12) by the second illumination device (20).

10. Method (100) according to any one of the claims 8 to 9, **characterized in that** the method (100) further comprising the step of:
enabling (460) together the first camera device (14) and the first illumination device (16) while disabling together the second camera device (18) and the second illumination device (20).

11. Method (100) according to claim 10, **characterized in that** the method (100) further comprising the step of:
enabling (480) together the second camera device (18) and the second illumination device (20) while disabling together the first camera device (14) and the first illumination device (16).

12. Method (100) according to any one of the claims 8 to 11, **characterized in that** the method (100) further comprises the steps of:
arranging (410) the first camera assembly (22) and the second camera assembly (24) with the front panel instrument (32) of the driver compartment (30) of the motor vehicle;
orienting (420) the first main optical axis (O₁) and the first main light axis (L₁) such that the first main optical axis (O₁) and the first main light axis (L₁) globally intersect at the driver's face;
orienting (430) the second main optical axis (O₂) and the second main light axis (L₂) such that the second main optical axis (O₂) and the second main light axis (L₂) globally intersect at the driver's face.

13. Method (100) according to any one of the claims 8 to 12, **characterized in that** the method (100) further comprises the step of:
orienting (440) the first main optical axis (O₁) and the first main light axis (L₁) such that the first main optical axis (O₁) and the first main light axis (L₁) intersect at a first angle (α) greater than 5°, preferably greater than 10°;
orienting (450) the second main optical axis (O₂) and the second main light axis (L₂), such that the second main optical axis (O₂) and the second main light axis (L₂) intersect at a second angle (β) greater than 5°, preferably greater than 10°.

14. Method (100) according to any one of the claims 8 to 13, **characterized in that** the method (100) further comprises the step of:
providing (210) the first camera device (14) and the second illumination device (20) distanced by a first distance (l₁) less than 40 millimeters, preferably less than 35 millimeters.

15. Method (100) according to any one of the claims 8 to 14, **characterized in that** the method (100) further comprises the step of:
providing (310) the second camera device (18) and the first illumination device (16) distanced by a second distance (l₂) less than 40 millimeters, preferably less than 35 millimeters.

## Patentansprüche

1. Fahrerüberwachungssystem (10) für ein Kraftfahrzeug, das konfiguriert ist, um Pupillen eines Fahrers (12) des Kraftfahrzeugs zu verfolgen, wobei das System (10) Folgendes umfasst:
eine erste Kameravorrichtung (14), die eine erste optische Hauptachse (O₁) umfasst, wobei die erste Kameravorrichtung (14) konfiguriert ist, um einen ersten Satz von Bildern der Pupillen des Fahrers (12) aufzunehmen;
eine erste Beleuchtungsvorrichtung (16), die eine erste Hauptlichtachse (L₁) umfasst, wobei die erste Beleuchtungsvorrichtung (16) konfiguriert ist, um die Pupillen des Fahrers (12) während eines Aufnahmevorgangs des ersten Satzes von Bildern zu beleuchten;
eine zweite Kameravorrichtung (18), die eine zweite optische Hauptachse (O₂) umfasst, wobei die zweite Kameravorrichtung (18) konfiguriert ist, um einen zweiten Satz von Bildern der Pupillen des Fahrers (12) aufzunehmen;
eine zweite Beleuchtungsvorrichtung (20), die eine zweite Hauptlichtachse (L₂) umfasst, wobei die zweite Beleuchtungsvorrichtung (20) konfiguriert ist, um die Pupillen des Fahrers (12) während eines Aufnahmevorgangs des zweiten Satzes von Bildern zu beleuchten;
**dadurch gekennzeichnet, dass** das System (10) ferner Folgendes umfasst:
eine erste Kamerabaugruppe (22), die die erste Kameravorrichtung (14) und die zweite Beleuchtungsvorrichtung (20) umfasst, wobei die erste Kameravorrichtung (14) physisch mit der zweiten Beleuchtungsvorrichtung (20) zusammengebaut ist;
eine zweite Kamerabaugruppe (24), die die zweite Kameravorrichtung (18) und die erste Beleuchtungsvorrichtung (16) umfasst, wobei die zweite Kameravorrichtung (18) physisch mit der ersten Beleuchtungsvorrichtung (16) zusammengebaut ist.

2. System (10) nach Anspruch 1, **dadurch gekennzeichnet, dass** das System (10) ferner eine elektronische Steuereinheit (26) in elektrischer Kommunikation mit der ersten Kamerabaugruppe (22) und mit der zweiten Kamerabaugruppe (24) umfasst, wobei die elektronische Steuereinheit (26) ein Schaltmittel (28) zum gemeinsamen Aktivieren der ersten Kameravorrichtung (14) und der ersten Beleuchtungsvorrichtung (16), während die zweite Kameravorrichtung (18) und die zweite Beleuchtungsvorrichtung (20) gemeinsam deaktiviert sind, umfasst.

3. System (10) nach einem der vorhergehenden Ansprüche, **dadurch gekennzeichnet, dass** die erste Kameravorrichtung (14) und die zweite Beleuchtungsvorrichtung (20) voneinander in einem ersten Abstand (l₁) von weniger als 40 Millimetern, vorzugsweise weniger als 35 Millimetern, angeordnet sind.

4. System (10) nach Anspruch 3, **dadurch gekennzeichnet, dass** die zweite Kameravorrichtung (18) und die erste Beleuchtungsvorrichtung (16) voneinander in einem zweiten Abstand (l₂) von weniger als 40 Millimetern, vorzugsweise weniger als 35 Millimetern, angeordnet sind.

5. System (10) nach einem der vorhergehenden Ansprüche, **dadurch gekennzeichnet, dass** sich die erste optische Hauptachse (O₁) und die erste Hauptlichtachse (L₁) in einem ersten Winkel (α) von mehr als 5°, vorzugsweise mehr als 10°, schneiden; und sich die zweite optische Hauptachse (O₂) und die zweite Hauptlichtachse (L₂) in einem zweiten Winkel (β) von mehr als 5°, vorzugsweise mehr als 10°, schneiden.

6. Fahrzeugführerabteil (30), das das System (10) nach einem der vorhergehenden Ansprüche umfasst, wobei das Fahrzeugführerabteil (30) Folgendes umfasst:
eine Frontplatteninstrumentenanordnung (32), die einen Armaturenbrettabschnitt (34) umfasst, der dem Fahrersitz des Fahrzeugs zugewandt ist,
**dadurch gekennzeichnet, dass**
die erste Kamerabaugruppe (22) und die zweite Kamerabaugruppe (24) mit dem Frontplatteninstrument (32) angeordnet sind;
sich die erste optische Hauptachse (O₁) und die erste Hauptlichtachse (L₁) im Kopfbereich des Fahrers des Fahrersitzes schneiden;
sich die zweite optische Hauptachse (O₂) und die zweite Hauptlichtachse (L₂) im Kopfbereich des Fahrers des Fahrersitzes schneiden.

7. Fahrzeugführerabteil (30) nach Anspruch 6, **dadurch gekennzeichnet, dass** die erste Kamerabaugruppe (22) innerhalb des Armaturenbrettabschnitts (34) angeordnet ist.

8. Verfahren (100) zum Betreiben des Fahrerüberwachungssystems (10) für ein Kraftfahrzeug nach einem der Ansprüche 1 bis 5, wobei das Verfahren (100) die folgenden Schritte umfasst:
Bereitstellen (200) einer ersten Kamerabaugruppe (22), die eine erste Kameravorrichtung (14) und eine zweite Beleuchtungsvorrichtung (20) umfasst;
Bereitstellen (300) einer zweiten Kamerabaugruppe (24), die eine zweite Kameravorrichtung (18) und eine erste Beleuchtungsvorrichtung (16) umfasst;
Aufnehmen (470) eines ersten Satzes von Bildern der Pupillen des Fahrers (12) mit der ersten Kameravorrichtung (14), während die Pupillen des Fahrers (12) durch die erste Beleuchtungsvorrichtung (16) beleuchtet werden.

9. Verfahren (100) nach Anspruch 8, **dadurch gekennzeichnet, dass** das Verfahren (100) ferner den folgenden Schritt umfasst:
Aufnehmen (490) eines zweiten Satzes von Bildern der Pupillen des Fahrers (12) mit der zweiten Kameravorrichtung (18), während die Pupillen des Fahrers (12) durch die zweite Beleuchtungsvorrichtung (20) beleuchtet werden.

10. Verfahren (100) nach einem der Ansprüche 8 bis 9, **dadurch gekennzeichnet, dass** das Verfahren (100) ferner den folgenden Schritt umfasst:
gemeinsames Aktivieren (460) der ersten Kameravorrichtung (14) und der ersten Beleuchtungsvorrichtung (16), während die zweite Kameravorrichtung (18) und die zweite Beleuchtungsvorrichtung (20) gemeinsam deaktiviert werden.

11. Verfahren (100) nach Anspruch 10, **dadurch gekennzeichnet, dass** das Verfahren (100) ferner den folgenden Schritt umfasst:
gemeinsames Aktivieren (480) der zweiten Kameravorrichtung (18) und der zweiten Beleuchtungsvorrichtung (20), während die erste Kameravorrichtung (14) und die erste Beleuchtungsvorrichtung (16) gemeinsam deaktiviert werden.

12. Verfahren (100) nach einem der Ansprüche 8 bis 11, **dadurch gekennzeichnet, dass** das Verfahren (100) ferner die folgenden Schritte umfasst:
Anordnen (410) der ersten Kamerabaugruppe (22) und der zweiten Kamerabaugruppe (24) mit dem Frontplatteninstrument (32) des Fahrerabteils (30) des Kraftfahrzeugs;
Ausrichten (420) der ersten optischen Hauptachse (O₁) und der ersten Hauptlichtachse (L₁) derart, dass sich die erste optische Hauptachse (O₁) und die erste Hauptlichtachse (L₁) global im Gesicht des Fahrers schneiden;
Ausrichten (430) der zweiten optischen Hauptachse (O₂) und der zweiten Hauptlichtachse (L₂) derart, dass sich die zweite optische Hauptachse (O₂) und die zweite Hauptlichtachse (L₂) global im Gesicht des Fahrers schneiden.

13. Verfahren (100) nach einem der Ansprüche 8 bis 12, **dadurch gekennzeichnet, dass** das Verfahren (100) ferner den folgenden Schritt umfasst:
Ausrichten (440) der ersten optischen Hauptachse (O₁) und der ersten Hauptlichtachse (L₁) derart, dass sich die erste optische Hauptachse (O₁) und die erste Hauptlichtachse (L₁) in einem ersten Winkel (α) von mehr als 5°, vorzugsweise mehr als 10°, schneiden;
Ausrichten (450) der zweiten optischen Hauptachse (O₂) und der zweiten Hauptlichtachse (L₂) derart, dass sich die zweite optische Hauptachse (O₂) und die zweite Hauptlichtachse (L₂) in einem zweiten Winkel (β) von mehr als 5°, vorzugsweise mehr als 10°, schneiden.

14. Verfahren (100) nach einem der Ansprüche 8 bis 13, **dadurch gekennzeichnet, dass** das Verfahren (100) ferner den folgenden Schritt umfasst:
Bereitstellen (210) der ersten Kameravorrichtung (14) und der zweiten Beleuchtungsvorrichtung (20), die um einen ersten Abstand (I₁) von weniger als 40 Millimetern, vorzugsweise weniger als 35 Millimetern, beabstandet sind.

15. Verfahren (100) nach einem der Ansprüche 8 bis 14, **dadurch gekennzeichnet, dass** das Verfahren (100) ferner den folgenden Schritt umfasst:
Bereitstellen (310) der zweiten Kameravorrichtung (18) und der ersten Beleuchtungsvorrichtung (16), die um einen zweiten Abstand (I₂) von weniger als 40 Millimetern, vorzugsweise weniger als 35 Millimetern, beabstandet sind.

## Revendications

1. Système de surveillance de conducteur (10) pour un véhicule motorisé configuré pour suivre les pupilles d'un conducteur (12) du véhicule motorisé, ledit système (10) comprenant
un premier dispositif de caméra (14) incluant un premier axe optique principal (O₁), ledit premier dispositif de caméra (14) étant configuré pour capturer une première série d'images des pupilles du conducteur (12) ;
un premier dispositif d'illumination (16) incluant un premier axe de lumière principal (L₁), ledit premier dispositif d'illumination (16) étant configuré pour illuminer les pupilles du conducteur (12) pendant une opération de capture de la première série d'images ;
un second dispositif de caméra (18) incluant un second axe optique principal (O₂), ledit second dispositif de caméra (18) étant configuré pour capturer une seconde série d'images des pupilles du conducteur (12) ;
un second dispositif d'illumination (20) incluant un second axe de lumière principal (L₂), ledit second dispositif d'illumination (20) étant configuré pour illuminer les pupilles du conducteur (12) pendant une opération de capture de la seconde série d'images ;
**caractérisé en ce que** le système (10) comprend en outre un premier ensemble formant caméra (22) comprenant le premier dispositif de caméra (14) et le second dispositif d'illumination (20), le premier dispositif de caméra (14) étant assemblé physiquement au second dispositif d'illumination (20) ;
un second ensemble formant caméra (24) comprenant le second dispositif de caméra (18) et le premier dispositif d'illumination (16), le second dispositif de caméra (18) étant assemblé physiquement au premier dispositif d'illumination (16).

2. Système (10) selon la revendication 1, **caractérisé en ce que** le système (10) comprend en outre une unité de commande électronique (26) en communication électrique avec le premier ensemble formant caméra (22) et le second ensemble formant caméra (24), ladite unité de commande électronique (26) comprenant un moyen de commutation (28) permettant d'activité conjointement le premier dispositif de caméra (14) et le premier dispositif d'illumination (16) tout en désactivant conjointement le second dispositif de caméra (18) et le second dispositif d'illumination (20).

3. Système (10) selon l'une quelconque des revendications précédentes, **caractérisé en ce que** le premier dispositif de caméra (14) et le second dispositif d'illumination (20) sont agencés l'un par rapport à l'autre à une première distance (I₁) qui est inférieure à 40 millimètres, de préférence inférieure à 35 millimètres.

4. Système (10) selon la revendication 3, **caractérisé en ce que** le second dispositif de caméra (18) et le premier dispositif d'illumination (16) sont agencés l'un par rapport à l'autre à une seconde distance (I₂) qui est inférieure à 40 millimètres, de préférence inférieure à 35 mm.

5. Système (10) selon l'une quelconque des revendications précédentes, **caractérisé en ce que** le premier axe optique principal (O₁) et le premier axe de lumière principal (L₁) se recoupent sous un premier angle (α) supérieur à 5°, de préférence supérieur à 10° ; et le second axe optique principal (O₂) et le second axe de lumière principal (L₂) se recoupent sous un second angle (β) supérieur à 5°, de préférence supérieur à 10°.

6. Habitacle de conducteur de véhicule (30) comprenant le système (10) selon l'une quelconque des revendications précédentes, ledit habitacle de conducteur de véhicule (30) comprenant
un ensemble formant instrumentation de panneau avant (32) comprenant une portion tableau de bord (34) faisant face au siège du conducteur du véhicule,
**caractérisé en ce que**
le premier ensemble formant caméra (22) et le second ensemble formant caméra (24) sont agencés avec l'instrumentation de panneau avant (32) ;
le premier axe optique principal (O₁) et le premier axe de lumière principal (L1) se recoupent au niveau de la zone de tête du conducteur du siège du conducteur ;
le second axe optique principal (O₂) et le second axe de lumière principal (L₂) se recoupent au niveau de la zone de tête du conducteur du siège du conducteur.

7. Habitacle de conducteur de véhicule (30) selon la revendication 6, **caractérisé en ce que** le premier ensemble formant caméra (22) est agencé à l'intérieur de la portion tableau de bord (34).

8. Procédé (100) de fonctionnement du système de surveillance de conducteur (10) destiné à un véhicule motorisé selon l'une quelconque des revendications 1 à 5, ledit procédé (100) comprenant les étapes consistant à :
fournir (200) un premier ensemble formant caméra (22) comprenant un premier dispositif de caméra (14) et un second dispositif d'illumination (20) ;
fournir (300) un second ensemble formant caméra (24) comprenant un second dispositif caméra (18) et un premier dispositif d'illumination (16) ;
capturer (470) une première série d'images des pupilles du conducteur (12) avec le premier dispositif de caméra (14) tout en illuminant les pupilles du conducteur (12) via le premier dispositif d'illumination (16).

9. Procédé (100) selon la revendication 8, **caractérisé en ce que** le procédé (100) comprend en outre l'étape consistant à :
capturer (490) une seconde série d'images des pupilles du conducteur (12) avec le second dispositif de caméra (18) tout en illuminant les pupilles du conducteur (12) via le second dispositif d'illumination (20)

10. Procédé (100) selon l'une quelconque des revendications 8 à 9, **caractérisé en ce que** le procédé (100) comprend en outre l'étape consistant à :
activer (460) conjointement le premier dispositif de caméra (14) et le premier dispositif d'illumination (16) tout en désactivant conjointement le second dispositif de caméra (18) et le second dispositif d'illumination (20).

11. Procédé (100) selon la revendication 10, **caractérisé en ce que** le procédé (100) comprend en outre l'étape consistant à :
activer (480) conjointement le second dispositif de caméra (18) et le second dispositif d'illumination (20) tout en désactivant conjointement le premier dispositif de caméra (14) et le premier dispositif d'illumination (16).

12. Procédé (100) selon l'une quelconque des revendications 8 à 11, **caractérisé en ce que** le procédé comprend en outre les étapes consistant à :
agencer (410) le premier ensemble formant caméra (22) et le second ensemble formant caméra (24) avec l'instrumentation de panneau avant (32) de l'habitacle de conducteur (30) du véhicule motorisé ;
orienter (420) le premier axe optique principal (O₁) et le premier axe de lumière principal (L₁) de telle sorte que le premier axe optique principal (O₁) et le premier axe de lumière principal (L₁) se recoupent globalement au niveau du visage du conducteur ;
orienter (430) le second axe optique principal (O₂) et le second axe de lumière principal (L₂) de telle sorte que le second axe optique principal (O₂) et le second axe de lumière principal (L₂) se recoupent globalement au niveau du visage du conducteur.

13. Procédé (100) selon l'une quelconque des revendications 8 à 12, **caractérisé en ce que** le procédé (100) comprend en outre les étapes consistant à :
orienter (440) le premier axe optique principal (O₁) et le premier axe de lumière principal (L₁) de telle sorte que le premier axe optique principal (O₁) et le premier axe de lumière principal (L₁) se recoupent sous un premier angle (α) supérieur à 5°, de préférence supérieur à 10° ;
orienter (450) le second axe optique principal (O₂) et le second axe de lumière principal (L₂) de telle sorte que le second axe optique principal (O₂) et le second axe de lumière principal (L₂) se recoupent et le second axe optique principal (O₂) et le second axe de lumière principal (L₂) se recoupent sous un second angle (β) supérieur à 5°, de préférence supérieur à 10°.

14. Procédé (100) selon l'une quelconque des revendications 8 à 13, **caractérisé en ce que** le procédé (100) comprend en outre l'étape consistant à :
fournir (210) le premier dispositif de caméra (14) et le second dispositif d'illumination (20) espacés à raison d'une première distance (I₁) qui est inférieure à 40 millimètres, de préférence inférieure à 35 millimètres.

15. Procédé (100) selon l'une quelconque des revendications 8 à 14, **caractérisé en ce que** le procédé (100) comprend en outre l'étape consistant à :
fournir (310) le second dispositif de caméra (18) et le premier dispositif d'illumination (16) espacés à raison d'une seconde distance (I₂) qui est inférieure à 40 millimètres, de préférence inférieure à 35 mm.
